# EUROPÄISCHE PATENTSCHRIFT

(11) **EP 1 360 245 B1**
(45) Veröffentlichungstag und Bekanntmachung des Hinweises auf die Patenterteilung: **14.07.2004**
(21) Anmeldenummer: 02735102.2
(22) Anmeldetag: 25.01.2002
(51) Int. Cl.: C09D 4/00, C09D 183/08, A61K 6/093

(54) **HAFTVERMITTLER FÜR METALL-POLYMER-VERBUNDE**
ADHESIVES FOR METAL-POLYMER COMPOSITES
AGENTS ADHESIFS POUR COMPOSITES METAL-POLYMERE

(30) Priorität: 26.01.2001 DE 10103586
(43) Veröffentlichungstag der Anmeldung: 12.11.2003
(73) Patentinhaber: 3M Espe AG, 82229 Seefeld (DE)
(72) Erfinder: GÖBEL, Roland, 07759 Jena (DE)
(74) Vertreter: Fiesser, Gerold Michael
(86) Internationale Anmeldenummer: PCT/EP2002/000798
(87) Internationale Veröffentlichungsnummer: WO 2002/072711

(56) Entgegenhaltungen:
- EP-A- 0 124 865
- US-A- 4 233 428
- US-A- 4 269 991

## Beschreibung

Die vorliegende Erfindung betrifft Haftvermittler für Metall-Polymer-Verbunde, die Alkoxysilane mit olefinisch ungesättigten Doppelbindungen und Alkoxysilane mit Mercaptogruppen enthalten, deren Verwendung und damit hergestellte Metall-Polymer-Verbunde.
Ästhetische Gestaltung und Funktionalität sind auf dem Gebiet der Zahnheilkunde untrennbar miteinander verbunden. Besonders die Gestaltung von Materialien zum Zahnersatz muss beiden Anforderungen in hohem Maße genügen, da der Zahnersatz einerseits die Funktionen des ursprünglich vorhandenen Zahnmaterials übernehmen soll, andererseits die äußere Erscheinung des Trägers des Zahnersatzmaterials sich zumindest nicht verschlechtem soll. Häufig werden dabei für Zahnersatzmaterialien als Grundmaterialien Metalle oder Metalllegierungen oder als "Dentallegierungen" bezeichnete Metallzusammensetzungen eingesetzt. Aufgrund der von der normalen Zahnfarbe abweichenden Färbung solcher Metalle oder Metalllegierungen wird deren optisches Erscheinungsbild im Mund jedoch vielfach als störend und unästhetisch empfunden.

Zur optimalen ästhetischen Gestaltung von Zahnersatz, beispielsweise von prothetischen Metallgerüsten wie Kronen oder Brücken, ist es daher notwendig, die Oberfläche der zu diesem Zweck eingesetzten Metalle oder Metalllegierungen mit einer zahnfarbenen Verblendung, beispielsweise einer Verblendung aus Kunststoff, zu versehen. An die Verbindung zwischen Metall und Verblendung werden jedoch hohe Anforderungen gestellt. So ist es beispielsweise besonders wichtig, dass die jeweiligen Verblendungen mit den Metallen spaltfrei und dauerhaft verbunden werden. Ein mangelhafter Verbund führt zum vorzeitigen Abplatzen der Verblendung oder zu Randspaltbildungen, die insbesondere bei Verblendungen aus Kunststoff gegebenenfalls zu einer Verfärbung des Randes durch Oxidation des Metallgerüstes und zu mechanischer Gebietsirritation am Spalt zwischen Verblendung und Metallgerüst führen kann.

Darüber hinaus unterliegt ein Metall-Kunststoff-Verbund, wie er bei Verblendungen aus Kunststoff vorliegt, im Mundmilieu besonderen Belastungen. Dies sind zum einen physikalisch-mechanische Belastungen wie sie bei den Kaubewegungen auftreten. Andererseits existieren im Mundmilieu auch chemische oder biologische Belastungen, beispielsweise durch Einfluss von Speichel, Nahrungsmitteln oder Medikamenten. Durch die im Mundmilieu zudem auftretenden Temperaturwechsel ist der Verbund weiterem mechanischen Stress ausgesetzt. Unter diesen extremen Bedingungen des Mundmilieus, der stetigen Feuchte- und Temperaturwechsel sowie der mechanischen Belastung des Verbundes erweisen sich bislang bekannte Typen von Verankerungen zur Befestigungen der Verblendung auf dem zu verblendenden Metall häufig als nicht in der Lage, einen auf Dauer ausreichend stabilen Verbund zwischen einer Dentallegierung und einem Verblendkunststoff aufrecht zu erhalten.

Das gebräuchliche Verfahren in der Zahntechnik zur Herstellung eines Verbundes zwischen Metallen und Kunststoffmaterialien war in der Vergangenheit beispielsweise der Einsatz von mechanischen Retentionen wie sie sich durch Aufbringen von Perlen oder Gittern auf dem Metall erhalten lassen. Nachteil dieses Systems sind zum einen der rein mechanische Verbund, der in den Randbereichen immer zu Spalten zwischen Metall und Kunststoffverblendung führt sowie der zusätzliche Platzbedarf für die Retentionen, der bei der anschließenden Verblendung die optische Gestaltung gravierend erschwert.

Bisherige Lösungswege ohne mechanische Retentionen führen entweder zu nicht genügend dauerhaften Verbindungen oder erforderten einen hohen, in zahntechnischen Labors häufig nicht akzeptablen, apparativen Aufwand.

Zum haftfesten, feuchtestabilen und spaltfreien Verbinden von Dentalkunststoffen mit Dentallegierungen wurden in den letzten Jahren zahlreiche Vorschläge gemacht. Grundprinzip vieler der vorgeschlagenen Verfahren ist es, dass in einem ersten Schritt eine silikatische Schicht auf die zu verblendende Metalloberfläche aufgebracht wird (Silikatisierung) und in einem weiteren Schritt diese Oberfläche mit einem funktionellen Alkoxysilan ausgerüstet wird (Silanisierung). Dabei wirkt dieses funktionelle Alkoxysilan als Bindeglied zwischen der auf der Metalloberfläche aufgebrachten anorganischen silikatischen Schicht und dem zur Verblendung eingesetzten Kunststoff. Dies wird dadurch erreicht, dass die OH-Gruppen des funktionellen Alkoxysilans mit den auf der Oberfläche der Silikatschicht vorhandenen OH-Gruppen im Rahmen einer Kondensationsreaktion kovalent verbunden werden, während andererseits eine weitere im funktionellen Alkoxysilan vorhandene funktionelle Gruppen mit dem zur Verblendung eingesetzten Kunststoff ebenfalls unter Ausbildung einer kovalenten Bindung reagieren kann.

So beschreibt beispielsweise die DE-C1 34 03 894 eine Vorrichtung zum Beschichten eines metallischen Dentalprothesenteils und ein Verfahren zum Betrieb einer solchen Vorrichtung. Dabei wird zum Beschichten eines metallischen Dentalprothesenteils mit einer Siliziumoxid enthaltenen Hanvermittlerschicht ein Flammhydrolysebrenner verwendet, der in einem Abstand von maximal 50 mm vom Dentalprothesenteil angeordnet ist. Dem Flammhydrolysebrenner wird dosiert eine in gasförmigem Aggregatzustand befindliche hydrolysierbare Siliziumverbindung zugeführt. Problematisch wirkt sich bei dem beschriebenen Verfahren aus, dass der apparative Aufwand außerordentlich hoch ist und darüber hinaus im Hinblick auf die Haftfestigkeit des Verbundes zwischen Metall und Verblendung Nachteile auftreten.

Die DE-C1 38 02 043 beschreibt ein Verfahren zur Vorbereitung einer Metalloberfläche für die Verbindung mit Kunststoff durch Aufbringen einer siliziumhaltigen Schicht unter Verwendung von siliziumhaltigem Material. Auf eine Metalloberfläche wird dabei durch Korundstrahlen mit einem Mittel enthaltend 0,1 bis 30 Gew.-% gegebenenfalls silanisiertes amorphes siliziumhaltiges Material mit einer Korngröße < 1 µm und als Rest ein Korundstrahlmedium mit einer mittleren Korngröße > 1 µm eine Schicht aufgebracht und diese gegebenenfalls anschließend silanisiert. Die Haftfestigkeit eines auf einer solchen Oberfläche erzeugten Verbundes ist jedoch nicht immer völlig befriedigend.

Aus der US-A 4,364,731 ist ein Verfahren zum Aufbringen einer Siliziumdioxidschicht auf metallische Dentalprothesenteile bekannt, bei dem eine Hochfrequenz-Magnetron-Sputter-Vorrichtung eingesetzt wird. Neben dem hohen apparativen Aufwand zur Durchführung des beschriebenen Verfahrens weisen die erhältlichen Metall-Polymer-Verbindungen den Nachteil auf, dass sie häufig keine ausreichenden Haftwerte zeigen.

Die DD-A1 276 453 beschreibt ein Verfahren zur Herstellung eines Metall/Kunststoff-Verbundkörpers, bei dem eine Silikat-Chromoxid-Schicht unter Zuhilfenahme einer Sol-Gel-Lösung auf die Oberfläche einer Dentallegierung aufgebracht und durch einen nachfolgenden Temperprozess verfestigt wird.

Insbesondere bei den beschriebenen Verfahren die den Hochfrequenz-Magnetron-Sputter, den Flammhydrolysebrenner und das Sol-Gel-Verfahren einsetzen, sind zur haftfesten Anbindung der Verbundschicht an die Legierungsoberfläche Temperaturen von etwa 300 °C erforderlich. Derart hohe Temperaturen wirken sich jedoch bei einer Vielzahl von Metalllegierungen nachteilig aus. So führt eine solche Temperaturbelastung beispielsweise bei Metalllegierungen mit einem Kupfergehalt von mehr als 5% zu Entmischungen an der Oberfläche der Legierung. Dies resultiert jedoch oft in Verfärbungen und in reduzierter Haftung im Hinblick auf eine Kunststoffverblendung.

Der Abstract der japanischen Patentanmeldung mit der Veröffentlichungsnummer JP 62292774 betrifft ein Verfahren, bei dem direkt auf die Oberfläche einer Legierung eine Vinylbenzyl-amino-triazin-dithiol, beispielsweise (10-Acryloyloxydesyloxy)-S-triazin-4,6-dithiol, enthaltende Grundierung aufgetragen wird.

Die DE-A1 198 57 367 beschreibt ein Mittel zur Behandlung einer Metalloberfläche, das jeweils mindestens 0,001Gew.-% eines polymerisierbaren Monomeren enthält. Ein polymerisierbares Monomeres weist dabei eine Thiophencarbonsäureestergruppe und ein weiteres polymerisierbares Monomeres weist eine Phosphorsäureestergruppe auf. Die Monomeren sind in einem organischen Lösemittel gelöst.

Nachteilig wirkt sich bei den in den beiden letztgenannten Druckschriften beschriebenen Verfahren aus, dass die beschriebenen Stoffe teilweise instabil sind. Darüber hinaus zeigen die mit Hilfe der oben beschriebenen Verbindungen hergestellten Metall-Polymer-Verbunde oft eine erhöhte Anfälligkeit gegenüber Hydrolyse und Oxidation. Darüber hinaus sind die beschriebenen Verbindungen in ihrer Herstellung aufwendig.

Es bestand daher ein Bedarf nach einem Mittel und einem Verfahren zur Herstellung yon unter den oben beschriebenen Umständen stabilen Metall-Polymer-Verbunden, welche die Nachteile des Standes der Technik vermeiden.

Der vorliegenden Erfindung lag daher die Aufgabe zugrunde ein Mittel zur Verfügung zu stellen, das unter Vermeidung von hohem apparativem Aufwand die Herstellung von hydrolysestabilen, haftfesten Metall-Polymer-Verbunden erlaubt. Weiterhin lag der Erfindung die Aufgabe zugrunde, ein Mittel zur Verfügung zu stellen, das die Herstellung von hydrolysestabilen, haftfesten Metall-Polymer-Verbunden erlaubt und darüber hinaus unter üblicherweise auftretenden Umgebungsbedingungen stabil ist.

Weiterhin lag der Erfindung die Aufgabe zugrunde ein Verfahren zur Herstellung von Metall-Polymer-Verbunden zur Verfügung zu stellen, das ohne hohen apparativen Aufwand zu hydrolysestabilen, haftfesten Metall-Polymer-Verbunden führt.

Die der Erfindung zugrundeliegenden Aufgaben werden durch Mittel und Verfahren gelöst, wie sie im nachfolgenden Text näher beschrieben werden. Gegenstand der vorliegenden Erfindung ist daher ein Haftvermittler für Metall-Polymer-Verbundstoffe, mindestens enthaltend ein mindestens eine ethylenisch ungesättigte Gruppe aufweisendes Alkoxysilan und mindestens ein mindestens eine Mercaptogruppe aufweisendes Alkoxysilan oder deren Kondensationsprodukte und mindestens ein organisches Lösemittel.

Unter dem Begriff "Haftvermittler" wird im Rahmen des vorliegenden Textes eine Zusammensetzung verstanden, welche die Haftung zwischen einer Metalloberfläche und einer mit einer solchen Metalloberfläche in Verbindung stehenden Polymerschicht verbessert. Der Begriff "verbessert" bezieht sich dabei auf den Vergleich der Haftung zwischen Metalloberfläche und Polymerschicht ohne Haftvermittler und mit Haftvermittler.

Unter einem "Metall-Polymer-Verbundstoff" wird im Rahmen der vorliegenden Erfindung ein Objekt verstanden, das mindestens eine Metalloberfläche aufweist, die mit mindestens einer Polymeroberfläche haftfest in Verbindung steht. Der Begriff "haftfest" bezieht sich dabei auf den Zustand, dass zwischen Metalloberfläche und Polymeroberfläche eine adhäsive Kraft wirkt.

Im Rahmen einer bevorzugten Ausführungsform der vorliegenden Erfindung handelt es sich bei einem "Metall-Polymer-Verbundstoff" wie er im Rahmen des vorliegenden Textes erwähnt wird um einen Verbundstoff, der im dentalmedizinischen Bereich eingesetzt werden kann.

Ein erfindungsgemäßer Haftvermittler eignet sich grundsätzlich zur Verbesserung der Adhäsion von Polymeren auf beliebigen Metallen. Geeignet sind beispielsweise die Metalle der 2. und 3. Hauptgruppe sowie der 1., 4., 5., 6., 7. und 8. Nebengruppe des Periodensystems der Elemente. Ebenfalls im Rahmen der vorliegenden Erfindung als Bestandteil der Metall-Polymer-Verbunde geeignet sind entsprechende Legierungen aus 2 oder mehr der genannten Metalle.

Als Metalle eignen sich beispielsweise Au, Ag, Pt, Pd, In, Cr, Co, Ti, Al, Mg, Mo, Fe, Ni, Cu, Zn oder Sn.

Im Rahmen einer weiteren Ausführungsform der vorliegenden Erfindung werden als Metalle Metalllegierungen eingesetzt, beispielsweise die kommerziell als sogenannte Dentallegierungen erhältlichen Metallverbindungen. Geeignete Legierungen enthalten beispielsweise Gemische aus zwei oder mehr der Metalle Au, Ag, Pd, Pt, Zn, In, Cu, Ti, Co oder Cr, Besonders geeignet sind beispielsweise Metalllegierungen mit einem Gehalt an Gold von mehr als etwa 50 Gew.-%, beispielsweise mehr als etwa 60, 70 oder 80 Gew.-%, wobei derartige Legierungen häufig noch einen Anteil an Kupfer von mehr als etwa 1 Gew.-%, beispielsweise etwa 2 bis etwa 12 Gew.-%, aufweisen können. Ebenfalls geeignet sind beispielsweise Legierungen mit einem hohen Gehalt an Co oder Cr oder beidem, beispielsweise Co/Cr-Legierungen.

Besonders geeignet sind dabei beispielsweise die unter den Namen Albabond® (Hersteller: Fa. Heraeus), Mainbond® EH (Hersteller: Fa. Heraeus), Galvanogold® (Hersteller: Fa. Wieland), Degunorm (Hersteller: Fa. Degussa), Wirobond C (Hersteller: Fa. BEGO) oder Wiron 88 (Hersteller: Fa. BEGO) erhältlichen Metalllegierungen.

Es hat sich im Rahmen der vorliegenden Erfindung gezeigt, dass der erfindungsgemäße Haftvermittler grundsätzlich auf einer im wesentlichen beliebigen Metallbasis eine deutliche Verbesserung der Hafteigenschaften eine Kunststoffverblendung auf der Metalloberfläche ergibt. Es ist jedoch im Rahmen der vorliegenden Erfindung bevorzugt, wenn der erfindungsgemäße Haftvermittler auf Metallen oder Metalllegierungen eingesetzt wird, die einen Gehalt an zu Komplexbildung befähigten Metallen in der Metallbasis aufweisen, beispielsweise auf Edelmetallen oder edelmetallhaltigen Legierungen. Besonders geeignete Legierungen enthalten beispielsweise mindestens eines oder ein Gemisch aus zwei oder mehr der Metalle Au, Ag, Pd, Pt, Co, Cr, Mo oder Ni.

Die Metallbasis kann grundsätzlich eine im wesentlichen beliebige Form und eine grundsätzlich im wesentlichen beliebige Oberflächenstruktur aufweisen. Es ist im Rahmen der vorliegenden Erfindung jedoch bevorzugt, wenn die Metallbasis eine aus dem im Rahmen der Dentalmedizin bekannten Formenschatz, wie er sich aus restaurativen und prothetischen Behandlungsmethoden ergibt, entnommene Form aufweist.

Ein erfindungsgemäßer Haftvermittler wird zur Herstellung eines Metall-Polymer-Verbundes eingesetzt. Die oben beschriebene Metallbasis wird dabei mit einer Polymerschicht versehen (verblendet) und soll zu dieser Polymerschicht eine möglichst starke Adhäsion aufweisen.

Bei einer Polymerschicht wie sie bei den im Rahmen des vorliegenden Textes beschriebenen Metall-Polymer-Verbunden vorzufinden ist, handelt es sich um ein Polymeres oder ein Gemisch aus zwei oder mehr Polymeren, wie sie durch radikalische Polymerisation herstellbar sind. Unter dem Begriff "durch radikalische Polymerisation herstellbar" wird im Rahmen des vorliegenden Textes der Befund verstanden, dass das abschließend im Metall-Polymer-Verbund vorliegende Polymere zumindest in einem letzten Schritt durch radikalische Polymerisation polymerisiert wurde. Dem steht nicht entgegen, dass das Polymere im Polymerrückgrat oder in gegebenenfalls vorhandenen Seitenketten oder in beidem Bindungen enthält, die durch eine anderer Methode zum Molekulargewichtsaufbau, beispielsweise durch Polyaddition oder Polykondensation, erzeugt wurden.

Vorzugsweise handelt es sich bei dem im Rahmen der vorliegenden Erfindung als Bestandteil des Metall-Polymer-Verbundes bezeichneten Polymeren um ein Polymeres, dessen abschließender Molekulargewichtsaufbau in Kontakt mit der Metallbasis beziehungsweise dem auf der Metallbasis befindlichen, im Rahmen des weiteren Textes näher beschriebenen Haftvermittlers, stattgefunden hat. Im Rahmen einer weiteren bevorzugten Ausführungsform der vorliegenden Erfindung handelt es sich bei dem im Rahmen der vorliegenden Erfindung als Bestandteil des Metall-Polymer-Verbundes bezeichneten Polymeren um ein Polymeres, dessen abschließender Molekulargewichtsaufbau durch radikalische Polymerisation radikalisch polymerisierbarer Verbindungen, insbesondere sogenannter Präpolymerer oder Makromonomerer, stattgefunden hat.

Als Präpolymere oder Makromonomere eignen sich hier beispielsweise Verbindungen mit mindestens einer, vorzugsweise mindestens zwei olefinisch ungesättigten Doppelbindungen. Besonders geeignet sind dabei Verbindungen, die sich durch Umsetzung entsprechender difunktioneller Verbindungen mit Acrylsäure oder Methacrylsäure oder anderen geeigneten Verbindungen mit mindestens einer olefinisch ungesättigten Doppelbindung erhalten lassen.

So eignen sich zur Herstellung der im Rahmen der vorliegenden Erfindung beschriebenen Polymeren beispielsweise Esteracrylate oder Estermethacrylate oder deren Gemische. Esteracrylate oder Estermethacrylate sind beispielsweise durch Umsetzung von Acrylsäure oder Methacrylsäure mit Verbindungen mit mindestens einer OH-Gruppe oder mindestens einer Epoxygruppe unter Kondensation oder Addition erhältlich. Als Verbindungen mit mindestens einer OH-Gruppe eignen sich grundsätzlich beliebige mono- oder polyfunktionelle Alkohole, vorzugsweise Alkohole mit zwei oder mehr, beispielsweise 2 bis 6, insbesondere 2 oder 3 OH-Gruppen. Geeignete Alkohole können sowohl aliphatischer als auch aromatischer Natur sein oder können beide Bausteine beinhalten.

Geeignet sind allgemein ein- und mehrfunktionelle (Meth)acrylatmonomere, wobei der Begriff "(Meth)acrylat" sowohl für Verbindungen auf Basis von Acrylsäure als auch für Verbindungen auf Basis von Methacrylsäure steht. Typische Vertreter dieser Verbindungsklasse (beispielsweise beschrieben in der DE-A-43 28 960) sind Alkyl(meth)acrylate, einschließlich der Cycloalkyl(meth)acrylate, Aralkyl(meth)acrylate und 2-Hydroxyalkyl(meth)acrylate, beispielsweise Hydroxypropylmethacrylat, Hydroxyethylmethacrylat, Isobomylacrylat, Isobornylmethacrylat, Butylglycolmethacrylat, Acetylglykolmethacrylat, Triethylenglycoldimethacrylat, Polyethylenglycoldimethacrylat, 2-Phenyiethylmethacrylat, 2-Ethylhexylmethacrylat, Cyclohexylmethacrylat, Laurylmethacrylat und Hexandioldi(meth)acrylat. Verwendet werden können auch langkettige Monomere auf der Basis von Bisphenol A und Glycidylmethacrylat, die aus der US-A-3 066 112 bekannt sind, oder deren durch Addition von Isocyanaten entstandenen Derivate (Urethanmethacrylate).

Geeignet sind auch Verbindungen des Typs Bisphenol-A-diethyloxy(meth)acrylat und Bisphenol-A-dipropyloxy(meth)acrylat. Weiterhin Verwendung finden können die oligoethoxylierten und oligopropoxylierten Bisphenol-A-diacryl- und -dimethacrysäureester. Gut geeignet sind außerdem die in der DE-C-28 16 823 genannten Diacryl- und Dimethacrylsäureester des Bis(hydroxymethyl)-tricyclo[5.2.1.0²⁶]-decans und die Diacryl- und Dimethacrylsäureester der mit 1 bis 3 Ethylenoxid- und/oder Propylenoxideinheiten verlängerten Verbindungen des Bis(hydroxymethyl)-tricyclo[5.2.1.0²⁶]-decans.

Die Herstellung entsprechender Esteracrylate oder Estermethacrylate ist dem Fachmann allgemeinen bekannt.

Ebenfalls als Präpolymere geeignet sind beispielsweise die bereits oben genannten mehrfunktionellen Urethanmethacrylate oder mehrfunktionellen Urethanacrylate.

Urethanmethacrylate sind durch Umsetzung von Polyisocyanaten mit OH-funktionellen Acrylaten oder Methacrylaten wie Hydroxyethylacrylat, Hydroxypropylacrylat Hydroxyethylmethacrylat oder Hydroxypropylmethacrylat erhältlich. Wird als Polyisocyanat einen Diisocyanat eingesetzt, so erhält man als Produkt ein Urethandimethacrylat, bei Einsatz eines OH-funktionellen Acrylats erhält man analog ein difunktionelles Acrylat.

Urethanmethacrylate oder Urethanacrylate, weisen ausgezeichnete Werkstoffeigenschaften wie hohe Steifigkeit oder geringe Wasseraufnahme auf. Möglich ist auch der Einsatz eines aus einer Kombination von Triisocyanaten oder höherwertigeren Polyisocyanaten mit OH-funktionellen Acrylaten oder Methacrylaten hergestellten Monomeren, wobei derartige Urethanmethacrylate bzw. Urethanacrylate im Hinblick auf olefinisch ungesättigte Doppelbindungen eine Funktionalität von 3 oder höher aufweisen.

Geeignete Präpolymere bzw. Makromonomere weisen bei einer Temperatur von 20°C eine Viskosität 5 von 10³ bis 5 * 10⁴ mPas, insbesondere von 1*10⁴ mPas (gemessen mit Rheo Stress RS 100, Fa. Haake), auf, da so bei einem Einsatz dieser Verbindungen zur Herstellung entsprechender Metall-Polymer-Verbunde auch ohne Verdünnung der Präpolymeren bzw. Makromonomeren eine gute Konsistenz gegeben ist.

Geeignete Präpolymere bzw. Makromonomere weisen ein Molekulargewicht von etwa 400 bis etwa 2500 g/mol, beispielsweise etwa 450 bis etwa 1000 oder etwa 500 bis etwa 800 g/mol auf.

Eine zur Herstellung entsprechender Polymerer geeignete Zusammensetzung kann über die obengenannten Bestandteil hinaus noch einen oder mehrere Reaktivverdünner enthalten. Unter dem Begriff "Reaktivverdünner" werden dabei im Rahmen des vorliegenden Textes Verbindungen verstanden, die für die zur Polykondensation eingesetzte Masse als Lösungs- oder Verdünnungsmittel wirken und somit die Viskosität der Master verringern, die jedoch darüber hinaus bei der Polymerisation der Masse, beispielsweise bei der Polymerisation mit Hilfe von Licht, in die Polymermatrix einpolymerisiert werden. Dazu geeignet sind beispielsweise weitere Monomethacrylate, Dimethacrylate und Trimethacrylate.

So eignen sich beispielsweise Dimethacrylatcomonomere wie Triethylenglykoldimethacrylat ("TEGDM"), Ethylenglykoldimethacrylat, Tetramethylenglykoldimethacrylat, Trimethylolpropyltrimethacrylat, 1,6-Hexandioldimethacrylat und 1,3-Butandioldimethacrylat als zusätzliche Bestandteile der zu polymerisierenden Massen.

Weiterhin kann eine zur Polymerisation eingesetzte Polymermasse weitere Zusatzstoffe wie Füllstoffe, Photoinitiatorsysteme und Farbpigmente enthalten.

Als Füllstoffe sind in der Regel anorganische Füllstoffe einsetzbar. Beispielhaft genannt seien Quarz, gemahlene Gläser, Kieselgele sowie pyrogene Kieselsäuren und Fällungskieselsäuren oder deren Granulate. Bevorzugt werden röntgenopake Füllstoffe, zumindest teilweise, mit eingesetzt. Diese können beispielsweise röntgenopake Gläser sein, also Gläser, welche beispielsweise Strontium, Barium oder Lanthan enthalten (z.B. nach US-A-3 971 754) oder ein Teil 10 der Füllkörper besteht aus einem röntgenopaken Zusatz, wie beispielsweise Yttriumtrifluorid, Strontiumhexafluorozirkonat oder Fluoriden der Selten-Erdmetalle (z.B. nach EP-A-0 238 025). Zum besseren Einbau in die Polymermatrix ist es von Vorteil, die anorganischen Füllstoffe zu hydrophobieren. Geeignete Füllstoffe können daher hydrophobiert, beispielsweise silanisiert, sein. Durch Verwendung von silanisiertem Füllstoff kann neben der Erhöhung der Benetzbarkeit durch Verringerung des polaren Charakters sogar eine chemische Einbindung in die Polymermatrix erzielt werden. Ein solcher Füllstoff wirkt als zusätzlicher Vernetzer und erhöht die Steifigkeit.

Übliche Hydrophobierungsmittel sind Silane, beispielsweise Trimethoxymethacryloyloxypropylsilan oder Trimethoxyglycidylsilan.

Geeignete Füllkörper haben vorzugsweise eine mittlere Kornverteilung < etwa 2 µm, insbesondere < etwa 0,5 µm, sowie eine obere Korngrenze von etwa 15 µm, vorzugsweise etwa 7 µm und insbesondere etwa 2,5 µm.

Besonders bevorzugt werden Gemische von 5 bis 25 Gew.-% Füllstoffe mit einer mittleren Korngröße von 0,02 bis 0,06 µm und 65 bis 85 Gew.-% Füllstoffe mit einer mittleren Korngröße von 1 bis 5 µm verwendet.

Wenn eine im Rahmen des vorliegenden Textes als Bestandteil eines Metall-Polymer-Verbunds beschriebene Polymerschicht als sogenannter "Opaker" eingesetzt wird, so kann die zur Herstellung der Polymerschicht eingesetzte Polymermasse als Füllstoffe beispielsweise besonders stark deckende Pigmente, insbesondere Titandioxid enthalten.

Als Initiatoren zur Initiierung der Polymerisation werden vorzugsweise solche Systeme verwendet, die in einem geeigneten Zeitraum Radikale zu bilden vermögen. Bei einkomponentigen Massen werden hierfür Photoinitiatoren eingesetzt, die durch Bestrahlung mit UV- oder sichtbarem Licht die Polymerisationsreaktion auslösen können. Als Photoinitiatoren kann eine zur Polymerisation vorgesehene Polymermasse beispielsweise einzelne geeignete Photoinitiatoren enthalten. Es ist jedoch ebenso möglich, dass eine zur Polymerisation eingesetzte Polymermasse Initiatorsysteme enthält, die Photoinitiatoren und CoInitiatoren umfassen.

Vertreter solcher Photoinitiatoren sind beispielsweise Benzoinalkylether, Benzilketale, Acylphosphinoxide oder aliphatische und aromatische 1,2-Diketonverbindungen, beispielsweise Campherchinon, wobei die Lichtpolymerisation durch Zusatz von Aktivatoren, wie tertiären Aminen oder 5 organischen Phosphiten, in an sich bekannter Weise beschleunigt werden kann.

Geeignete Initiatorsysteme zur Auslösung der Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor ihrer Anwendung miteinander homogen vermischt.

Geeignete Hilfsstoffe können beispielsweise üblicherweise auf dem Dentalgebiet eingesetzte Stabilisatoren, Pigmente oder Verdünnungsmittel sein.

Die Herstellung der zur Polymerisation vorgesehenen Massen erfolgt vorzugsweise so, dass die flüssigen Bestandteile miteinander gemischt werden, die Initiatoren, sofern sie nicht flüssig sind, darin durch Rühren eingebracht werden und anschließend die Füllstoffe zugegeben werden. Eine gute Homogenisierung kann beispielsweise durch Kneten erreicht werden.

Zweikomponentige zur Polymerisation geeignete Massen, deren Aushärtung durch Redox-Mechanismen erfolgt, werden so formuliert, dass die wesentlichen Bestandteile des Redox-Initiierungssystems getrennt in je einem Teil der zweikomponentigen Zubereitung eingebracht werden. Die Aufteilung der Bestandteile der Gesamtzubereitung richtet sich nach den jeweiligen Lagerbeständigkeiten und dem angestrebten Mischungsverhältnis.

Die polymerisierbaren Massen zeichnen sich durch einen hohen Füllstoffanteil und damit verbundener hoher Festigkeit bei gleichzeitig guter Verarbeitbarkeit aus.

Die polymerisierbaren Massen sind üblicherweise kommerziell in Behältnissen wie Schlauchbeuteln, Ein- oder Mehrkammerkartuschen oder Kapseln und anderen Applikationseinheiten, beispielsweise Blisterverpackungen oder Spritzen, wie aus dem dentalen Bereich bekannt, erhältlich.

Weitere geeignete polymerisierbare Massen können darüber hinaus noch Siloxanverbindungen enthalten, wie sie beispielsweise in der WO 01/92271 beschrieben werden. Auf die Offenbarung der genannten Druckschrift wird ausdrücklich verwiesen und der die Siloxanverbindungen betreffende Teil der Offenbarung wird als Bestandteil der Offenbarung des vorliegenden Textes betrachtet.

Die Dicke einer Polymerschicht, wie sie im Rahmen der vorliegend beschriebenen Metall-Polymer-Verbunde auftritt, kann in weiten Grenzen variieren. Die Untergrenze für die Dicke einer geeigneten Polymerschicht liegt bei etwa 20 µm, während die Obergrenze bei etwa 5 mm liegt.

Ein im Rahmen des vorliegenden Textes beschriebener Metall-Polymer-Verbund wird erfindungsgemäß mit einem Haftvermittler hergestellt, der die Adhäsion zwischen Metall und Polymerschicht verbessert. Ein erfindungsgemäßer Haftvermittler enthält mindestens ein mindestens eine olefinisch ungesättigte Doppelbindung aufweisendes Alkoxysilan und mindestens ein mindestens eine Mercaptogruppe aufweisendes Alkoxysilan oder deren Kondensationsprodukte.

Unter einem "Alkoxysilan" wird im Rahmen der vorliegenden Erfindung eine Verbindung verstanden, die mindestens eine funktionelle Gruppe der allgemeinen Formel I 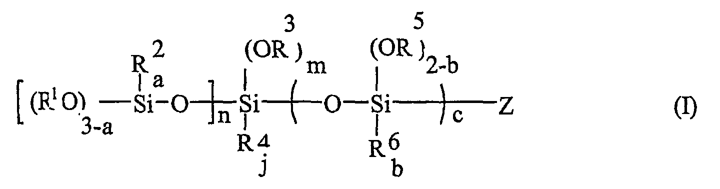 worin die Reste R¹ bis R⁶ jeweils unabhängig voneinander für einen linearen oder verzweigten, gesättigten oder ungesättigten Kohlenwasserstoffrest mit 1 bis etwa 24 C-Atomen, einen gesättigten oder ungesättigten Cycloalkylrest mit 4 bis etwa 24 C-Atomen oder einen Arylrest mit 6 bis etwa 24 C-Atomen stehen, n, m und j jeweils für eine ganze Zahl von 0 bis 3 stehen, wobei m + n + j = 3, a für eine ganze Zahl von 0 bis 3, b für eine ganze Zahl von 0 bis 2 und c für eine Zahl von 0 bis 8 und Z für einen mindestens eine olefinisch ungesättigte Doppelbindung oder mindestens eine Mercaptogruppe oder einen mindestens eine olefinisch ungesättigte Doppelbindung und mindestens eine Mercaptogruppe tragenden Rest steht.

Im Rahmen einer bevorzugten Ausführungsform der vorliegenden Erfindung stehen n, j und c jeweils für 0, und m für 3, wobei die Reste R³ unabhängig voneinander für CH₃ oder CH₃-CH₂ stehen.

Eine im Rahmen der vorliegenden Erfindung einsetzbares und als "Alkoxysilan" bezeichnete Verbindungen kann eine Alkoxysilylgruppe der allgemeinen Formel I oder mehrere Alkoxysilylgruppen der allgemeinen Formel I aufweisen. Im Rahmen einer bevorzugten Ausführungsform der vorliegenden Erfindung weist eine als Alkoxysilan bezeichnete Verbindungen 1 oder 2 Alkoxysilylgruppen der allgemeinen Formel I, vorzugsweise eine Alkoxysilylgruppe der allgemeinen Formel I auf.

Ein erfindungsgemäßer Haftvermittler enthält mindestens eine Verbindung die mindestens eine Alkoxysilylgruppe und einen Rest Z aufweist, der mindestens eine olefinisch ungesättigte Doppelbindung trägt. Der Rest Z kann dabei im wesentlichen beliebig gewählt werden, solange er mindestens eine olefinisch ungesättigte Doppelbindung trägt.

So sind beispielsweise Verbindungen geeignet, welche die olefinisch ungesättigte Doppelbindungen direkt über eine Kohlenstoffkette mit dem Si-Atom verknüpft tragen, beispielsweise Vinyltrimethoxysilan oder Vinyltriethoxysilan.

Beispielsweise handelt es sich bei dem Rest Z jedoch um einen Rest, der über mindestens eine Acryloyl oder eine Methacryloylgruppe trägt. Geeignete Reste Z sind beispielsweise Acryloyloxyethyl-, Acryloyloxypropyl-, Acryloyloxybutyl-, Acryloyloxypentyl- oder Acryloyloxyhexylreste oder Acryloyloxyalkylreste noch mit mehr als 6 C-Atomen, beispielsweise mit 7 bis 20 oder 8 bis 14 C-Atomen, oder die entsprechenden Methacryloylverbindungen. Die Acryloyl- oder Methacryloyloxyalkylreste können dabei linear oder verzweigt, vorzugsweise jedoch linear sein.

Erfindungsgemäß besonders geeignete Alkoxysilane mit mindestens einer olefinisch ungesättigten Doppelbindung sind Vinyltrimethoxysilan, Vinyltriethoxysilan, Trimethoxysilylethyl(meth)acrylat, Triethoxysilylethyl(meth)acrylat, Ethoxydimethoxysilylethyl(meth)acrylat, Methoxydiethoxysilylethyl(meth)acrylat, Trimethoxysilylpropyl(meth)acrylat, Triethoxysilylpropyl(meth)acrylat, Ethoxydimethoxysilylpropyl(meth)acrylat, Methoxydiethoxysilylpropyl(meth)acrylat, Trimethoxysilylbutyl(meth)acrylat, Triethoxysilylbutyl(meth)acrylat, Ethoxydimethoxysilylbutyl(meth)acrylat, Methoxydiethoxysilylbutyl(meth)-acrylat, Trimethoxysilylpentyl(meth)acrylat, Triethoxysilylpentyl(meth)acrylat, Ethoxydimethoxysilylpentyl(meth)acrylat, Methoxydiethoxysilylpentyl(meth)acrylat, Trimethoxysilylhexyl(meth)acrylat, Triethoxysilylhexyl(meth)acrylat, Ethoxydimethoxysilylhexyl(meth)acrylat oder Methoxydiethoxysilylhexyl(meth)acrylat oder Gemische aus zwei oder mehr davon.

Neben einem Alkoxysilan mit mindestens einer olefinisch ungesättigten Doppelbindung enthält ein erfindungsgemäßer Haftvermittler noch mindestens ein Alkoxysilan mit mindestens einer Mercaptogruppe. Geeignete Mercaptogruppen tragende Alkoxysilane folgen ebenfalls der allgemeinen Formel I, wobei der Rest Z mindestens eine Mercaptogruppe trägt.

So sind beispielsweise Verbindungen geeignet, welche die Mercaptogruppe über eine Kohlenstoffkette mit dem Si-Atom verknüpft tragen, beispielsweise Mercaptoethyltrimethoxysilan, Mercaptoethyltriethoxysilan, Mercaptopropyltrimethoxysilan, Mercaptopropyltriethoxysilan, Mercaptobutyltrimethoxysilan, Mercaptobutyltriethoxysilan, Mercaptopentyltrimethoxysilan, Mercaptopentyltriethoxysilan oder die entsprechenden höheren Homologen mit bis zu etwa 20 C-Atomen, beispielsweise etwa 6 bis etwa 12 C-Atomen, im Alkylenrest.

Besonders geeignet als Bestandteil eines erfindungsgemäßen Haftvermittlers sind im Rahmen der vorliegenden Erfindung Mercaptopropyltrimethoxysilan oder Mercaptopropyltriethoxysilan oder deren Gemisch.

Ein erfindungsgemäßer Haftvermittler enthält die beiden oben beschriebenen Komponenten, also ein Alkoxysilan mit mindestens einer olefinisch ungesättigten Doppelbindung und ein Alkoxysilan mit mindestens einer Mercaptogruppe in einer Menge von insgesamt bis zu etwa 70 Gew.-%, beispielsweise in einer Menge von etwa 0,1 bis etwa 10 oder etwa 0,2 bis etwa 5, beispielsweise etwa 0,4 bis etwa 3 oder etwa 0,7 bis etwa 2 Gew.-%, bezogen auf den gesamten Haftvermittler.

Das Verhältnis von Alkoxysilan mit mindestens einer olefinisch ungesättigten Doppelbindung zu Alkoxysilan mit mindestens einer Mercaptogruppe beträgt etwa 1:10 bis etwa 10:1, insbesondere etwa 1:2 bis etwa 2:1 oder etwa 1:1,2 bis etwa 1,2:1.

Es ist dabei im Rahmen der vorliegenden Erfindung möglich und auch vorgesehen, dass die beiden zwingend in einem erfindungsgemäßen Haftvermittler vorliegenden Bestandteile nicht in ihrer oben beschriebenen monomolekularen Form im Haftvermittler enthalten sind sondern als Kondensationsprodukt. Dies ist insbesondere dann der Fall, wenn ein erfindungsgemäßer Haftvermittler neben den beiden oben beschriebenen Verbindungstypen noch mindestens eine weitere Verbindung enthält, welche die Kondensation der im Haftvermittler enthaltenen Verbindungen mit Alkoxysilylgruppen initiiert oder beschleunigt. In einem solchen Fall kann es zu einer zumindest teilweisen Kondensation der im Haftvermittler enthaltenen Verbindungen mit Alkoxysilylgruppen kommen. Dies berührt jedoch die Wirksamkeit der erfindungsgemäßen Haftvermittler nicht.

Neben den beiden oben beschriebenen Verbindungstypen mit Alkoxysilylgruppen enthält ein erfindungsgemäßer Haftvermittler noch mindestens ein organisches Lösemittel. Als organisches Lösemittel geeignet sind grundsätzlich alle Lösemittel, welche die im erfindungsgemäßen Haftvermittler enthaltenen Verbindungen lösen und nach dem Auftrag des Haftvermittlers auf die Metalloberfläche ausreichend schnell verdunsten. Vorzugsweise handelt es sich dabei um Lösemittel mit einem Siedepunkt von weniger als etwa 100 °C. Geeignete Lösemittel sind beispielsweise Ethanol, Propanol, Isopropanol, Aceton, Methylethylketon, Essigsäuremethylester oder Essigsäureethylester oder Gemische aus zwei oder mehr davon.

Geeignete Lösemittel weisen einen Wassergehalt auf, der unterhalb von etwa 1 Gew.-%, insbesondere unterhalb von etwa 0,5 Gew.-% oder unterhalb von etwa 0,1 Gew.-% liegt.

Der Gehalt des erfindungsgemäßen Haftvermittlers an Lösemittel oder Lösemittelgemisch beträgt, bezogen auf den gesamten Haftvermittler etwa 70 bis etwa 99,9 Gew.-%, insbesondere etwa 85 bis etwa 99,5 Gew.-%

Neben den obengenannten Alkoxysilanen und einem organischen Lösemittel oder Lösemittelgemisch kann ein erfindungsgemäßer Haftvermittler noch eine Säure enthalten. Als Säuren eignen sich grundsätzlich anorganische Säuren wie Salzsäure, Salpetersäure, Schwefelsäure, Phosphorsäure oder phosphorige Säure. Es ist im Rahmen der vorliegenden Erfindung jedoch ebenso möglich, dass ein erfindungsgemäßer Haftvermittler eine organische Säure oder ein Gemisch aus zwei oder mehr organischen Säuren enthält. Grundsätzlich sind dabei als Säuren aller organischen Verbindungen geeignet, die im Haftvermittler eine saure Wirkung entfalten Besonders geeignet sind dabei organische Carbonsäuren oder Phosphonsäuren wie Essigsäure, Weinsäure oder Maleinsäure. Ebenfalls geeignet sind dabei diejenigen Verbindungen, die einerseits eine saure Wirkung entfalten und andererseits über eine entsprechende funktionelle Gruppe in die auf der Metalloberfläche aufzutragende Polymerschicht eingebaut werden können. Derartige Verbindungen werden im Rahmen des vorliegenden Textes als "saure Monomere" bezeichnet.

Besonders als saure Monomere geeignet sind hierbei Phosphorsäureester, die eine oder mehrere olefinisch ungesättigte Doppelbindungen enthalten. Derartige Verbindungen lassen sich beispielsweise aus OH-Gruppen tragenden Alkylphosphaten durch Umsetzung mit Carbonsäuren oder Epoxiden erhalten, die eine oder mehrere olefinisch ungesättigte Doppelbindungen aufweisen. Besonders als Bestandteil der erfindungsgemäßen Haftvermittler geeignet sind die Ester von Acrylsäure oder Methacrylsäure mit OH-Gruppen tragenden linearen oder verzweigten Alkylphosphaten wie Acryloyloxyethylphosphat, Acryloyloxypropylphosphat, Acryloyloxybutylphosphat, Acryloyloxypentylphosphat, Acryloyloxyhexylphosphat, Methacryloyloxyethylphosphat, Methacryloyloxypropylphosphat, Methacryloyloxybutylphosphat, Methacryloyloxypentylphosphat, Methacryloyloxyhexylphosphat oder die entsprechenden höheren Homologen mit bis zu 20 C-Atomen, beispielsweise etwa 7 bis etwa 10 oder etwa 12 C-Atomen, in der Kohlenstoffkette, etwa Methacryloyloxydecylphosphat.

Die obengenannten Säuren können im Rahmen eines erfindungsgemäßen Haftvermittlers jeweils alleine oder als Gemisch aus zwei oder mehr der genannten Säuren vorliegen.

Wenn ein erfindungsgemäßer Haftvermittler eine Säure oder ein Gemisch aus zwei oder mehr Säuren enthält, so beträgt der Gehalt des Haftvermittlers an Säure oder Säuren etwa 0,5 bis etwa 25 Gew.-%, insbesondere etwa 1 bis etwa 10 oder etwa 2 bis etwa 8 Gew.-%.

Gegebenenfalls kann ein erfindungsgemäßer Haftvermittler neben den bereits beschriebenen Verbindungstypen noch einen oder mehrere Zusatzstoffe enthalten. Geeignete Zusatzstoffe sind beispielsweise Vernetzer.

Als Vernetzer eignen sich beispielsweise Verbindungen, die zwei oder mehr olefinisch ungesättigte Doppelbindungen aufweisen. Besonders geeignet sind dabei die bereits oben im Rahmen des vorliegenden Textes bereits beschriebenen Ester von zwei- oder mehrwertigen Alkoholen mit Acrylsäure oder Methacrylsäure wie Ethylenglycoldi(meth)acrylat, Propylenglycoldi(meth)acrylat, Butandioldi(meth)acrylat, Hexandioldi(meth)acrylat, Triethylenglycoldi(meth)acrylat, Tetraethylenglycoldi(meth)acrylat, Neopentylglycoldi(meth)acrylat, Trimethylolpropantri(meth)acrylat oder (Meth)acrylatester aromatischer polyfunktioneller Alkohole, insbesondere die (Meth)acrylatester von Bisphenol-A. Ein erfindungsgemäßer Haftvermittler kann die genannten Vernetzer dabei jeweils einzeln oder als Gemisch aus zwei oder mehr der genannten Vernetzer enthalten. Der Gehalt eines erfindungsgemäßen Haftvermittlers an Vernetzer oder Vernetzern beträgt etwa 0,1 bis etwa 30 Gew.-%, insbesondere etwa 0,5 bis etwa 20 oder etwa 1 bis etwa 15 oder etwa 2 bis etwa 20 Gew.-%. Ein erfindungsgemäßer Haftvermittler weist im Rahmen einer bevorzugten Ausführungsform der vorliegenden Erfindung beispielsweise folgende Zusammensetzung auf:
- 0,1 bis 20 Gew.-% eines Alkoxysilans mit mindestens einer olefinisch ungesättigten Doppelbindung
- 0,1 bis 10 Gew.-% eines Alkoxysilans mit einer Mercaptogruppe
- 0 bis 10 Gew.-% Vernetzer
- 0,1 bis 20 Gew.-% Säure oder saure Monomere und
- 79,8 bis 99,7 Gew.-% Lösemittel.

Eine als Haftvermittler geeignete Rezeptur umfasst beispielsweise 5 Gew.-% γ-Methacryloyloxypropyltrimethoxysilan, 2 Gew.-% ml Trimethoxysilyl-propanthiol, 0,5 Gew.-% 0,1 n HCl und 92,5 Gew.-% Aceton.

Ein erfindungsgemäßer Haftvermittler kann, wie bereits oben beschrieben, die zwingend sowie die optional im Haftvermittler vorliegenden Komponenten in Form einer einzigen Lösungen zur Verfügung stellen. Es ist jedoch im Rahmen der vorliegenden Erfindung ebenfalls vorgesehen, dass ein erfindungsgemäßer Haftvermittler die zum Aufbau der haftvermittelnden Schicht benötigten Verbindungen in Form eines Kits umfassend zwei oder mehr voneinander getrennt vorliegende Komponenten (Lösungen) zur Verfügung gestellt wird.

Es hat sich dabei grundsätzlich bewährt, wenn der Haftvermittler aus mindestens zwei Komponenten A und B besteht, wobei Komponente A mindestens ein Alkoxysilan, vorzugsweise jedoch alle im Haftvermittler enthaltenen Alkoxysilane und Komponente B eine Säure oder ein saures Monomeres enthält.

Gegenstand der vorliegenden Erfindung ist daher auch ein Haftvermittler, mindestens umfassend zwei voneinander getrennt vorliegende Komponenten A und B, wobei
a) Komponente A mindestens ein mindestens eine olefinisch ungesättigte Doppelbindung aufweisendes Alkoxysilan und mindestens ein mindestens eine Mercaptogruppe aufweisendes Alkoxysilan oder deren Kondensationsprodukte und
b) Komponente B mindestens eine Säure oder mindestens ein saures Monomeres oder ein Gemisch aus zwei oder mehr davon enthält.

Der Begriff "voneinander getrennt vorliegen" umfasst im Rahmen der vorliegenden Erfindung den Befund, dass die zum erfindungsgemäßen in mehreren Komponenten vorliegenden Haftvermittler gehörenden Komponenten derart räumlich voneinander getrennt sind, dass keine Vermischung der einzelnen Komponenten stattfinden kann. Dies kann beispielsweise die Lagerung in räumlich voneinander getrennten Gebinden, beispielsweise die Lagerung in Flaschen, bedeuten. Es ist jedoch ebenso möglich, dass die unterschiedlichen Komponenten in einem einzigen Gebinde gelagert werden, das beispielsweise über verschiedene durch Trennwände voneinander separierte Kammern verfügt und die einzelnen Komponenten des erfindungsgemäßen Haftvermittlers in den jeweiligen Kammern gelagert werden.

Die Abgabeform eines erfindungsgemäßen mehrkomponentigen Haftvermittlers kann dabei derart erfolgen, dass die einzelnen Komponenten jeweils frei erhältlich sind. Es ist jedoch im Rahmen der vorliegenden Erfindung ebenso vorgesehen, dass die einzelnen Komponenten eines erfindungsgemäßen mehrkomponentigen Haftvermittlers zusammen, beispielsweise in einem entsprechenden über mehrere Kammern verfügenden Gebinde oder in getrennten Gebinden die beispielsweise im Rahmen einer geeigneten Verpackung, etwa einer Blisterverpackung, miteinander verbunden sind, abgegeben werden.

Im Rahmen einer bevorzugten Ausführungsform der vorliegenden Erfindung umfasst ein erfindungsgemäßer mehrkomponentiger Haftvermittler zwei Komponenten A und B, wobei Komponente A die im Rahmen des erfindungsgemäßen Haftvermittlers vorliegenden Alkoxysilan und Komponente B eine Säure oder ein saures Monomeres oder ein Gemisch davon enthält.

Im Rahmen einer weiteren bevorzugten Ausführungsform der vorliegenden Erfindung enthält Komponente A ein Lösemittel oder ein Gemisch aus zwei oder mehr Lösemitteln. Als Lösemittel eignen sich die bereits im Rahmen des vorliegenden Textes oben genannten Lösemittel. Es ist für die Lagerstabilität des erfindungsgemäßen Haftvermittlers vorteilhaft, wenn dass in Komponente A vorliegende Lösemittel einen Wassergehalt von weniger als etwa 0,1 Gew.-%, insbesondere von weniger als etwa 0,05 Gew.-% aufweist.

Im Rahmen einer weiteren bevorzugten Ausführungsform der vorliegenden Erfindung enthält eine geeignete Komponente A beispielsweise
- etwa 0,5 bis etwa 30 Gew.-% mindestens eines Alkoxysilans mit einer olefinisch ungesättigten Doppelbindung
- etwa 0,5 bis etwa 20 Gew.-% mindestens eines Alkoxysilans mit mindestens einer Mercaptogruppe und
- etwa 50 bis etwa 99 Gew.-% eines Lösemittels oder eines Lösemittelgemischs.

Entsprechende als Komponente B eines erfindungsgemäßen Haftvermittlers geeignete Zusammensetzungen enthaltend mindestens eine Säure oder ein saures Monomeres. Im Rahmen einer bevorzugten Ausführungsform der vorliegenden Erfindung enthält eine als Komponente B geeignete Zusammensetzung noch mindestens ein Lösemittel. Eine geeignete Komponente B kann beispielsweise die folgende Zusammensetzung aufweisen:
- etwa 0,5 bis etwa 50 Gew.-%, insbesondere etwa 5 bis etwa 40 Gew.-% einer Säure oder eines sauren Monomeren und
- etwa 50 bis etwa 99,5 Gew.-%, insbesondere etwa 60 bis etwa 80 Gew.-% eines Lösemittels oder eines Lösemittelgemischs.

Die im Rahmen der vorliegenden Erfindung beschriebenen Haftvermittler eignen sich zur Herstellung von Metall-Polymer-Verbunden.

Grundsätzlich wird dabei eine Metalloberfläche mit einem erfindungsgemäßen Haftvermittler versehen. Gegenstand der vorliegenden Erfindung ist daher auch ein Verfahren zur Behandlung einer Metalloberfläche, bei dem eine Metalloberfläche mit einem erfindungsgemäßen Haftvermittler versehen wird.

Das erfindungsgemäße Verfahren kann grundsätzlich auf beliebige Weise durchgeführt werden, solange nach Abschluss des Verfahrens eine Schicht eines erfindungsgemäßen Haftvermittlers auf der Metalloberfläche vorliegt. Wird beispielsweise im Rahmen des erfindungsgemäßen Verfahrens ein einkomponentiger Haftvermittler eingesetzt, so lässt sich das erfindungsgemäße Verfahren dahingehend Durchführung, dass der Haftvermittler in einem oder mehreren Arbeitsgängen auf die Metalloberfläche aufgetragen wird. Vorzugsweise findet der Auftrag in einem Arbeitsgang statt.

Zum Auftragen eignen sich grundsätzlich alle Auftragsmethoden, mit denen eine ausreichend dicke Schicht des Haftvermittlers auf der Metalloberfläche aufgebracht werden kann. Geeignete Auftragmethoden sind beispielsweise Pinseln, Rollen, Rakeln oder Sprühen. Im Rahmen einer bevorzugten Ausführungsform der vorliegenden Erfindung wird zum Auftrag des erfindungsgemäßen Haftvermittlers auf die Metalloberfläche ein Pinsel eingesetzt. Nach dem Auftrag des Haftvermittlers auf die Metalloberfläche wird vorzugsweise solange gewartet, bis das im Haftvermittler enthaltene Lösemittel im wesentlichen weitgehend verdunstet ist, d. h., dass in der Schicht aus Haftvermittler nur noch höchstens etwa 5 Gew.-%, vorzugsweise jedoch weniger als etwa 1 Gew.-% des ursprünglich enthaltenen Lösemittels vorliegen

Wenn einer Metalloberfläche mit einem erfindungsgemäßen mehrkomponentigen Haftvermittler behandelt werden soll, so bieten sich hierzu verschiedene Verfahrensschritte an. So kann beispielsweise die Metalloberfläche zunächst mit der Komponente A und anschließend mit der Komponente B behandelt werden. Es ist jedoch auch eine umgekehrte Vorgehensweise möglich, d. h., dass die Metalloberfläche zunächst mit der Komponente B behandelt wird und erst anschließend mit der Komponente A. Dabei können zwischen den einzelnen Behandlungsschritten Trockenschritte eingefügt werden, bei denen das in den jeweiligen Komponenten enthaltene Lösemittel ganz oder teilweise verdunstet.

Es ist im Rahmen der vorliegenden Erfindung jedoch ebenso möglich, dass die Behandlung mit einem mehrkomponentigen erfindungsgemäßen Haftvermittler im wesentlichen wie für den einkomponentigen Haftvermittler beschrieben erfolgt. Dazu werden die einzelnen Komponenten des mehrkomponentigen Haftvermittlers vor dem Auftrag auf die Metalloberfläche im gewünschten Verhältnis vermischt und die Mischung auf die Metalloberfläche aufgetragen.

Aus den so behandelten Metalloberflächen lassen sich anschließend die bereits beschriebenen Metall-Polymer-Verbunde herstellen. Hierzu wird eine polymerisierbare Masse wie sie bereits oben im Rahmen des vorliegenden Textes beschrieben wurde, auf die vorbehandelte Metalloberfläche aufgebracht und polymerisiert. Geeignete Vorgehensweisen sind dem Fachmann bekannt. Es ist im Rahmen der Erfindung dabei möglich, vor dem Auftrag der abschließenden Polymerschicht noch einen oder mehrere Zwischenschritte einzufügen, beispielsweise kann es bevorzugt sein, vor dem Auftrag der Polymerschicht noch einen Opaker, wie er üblicherweise im Dentalbereich eingesetzt wird und dem Fachmann bekannt ist, auf die mit dem Haftvermittler vorbehandelte Metalloberfläche aufzutragen und zu polymerisieren.

Unter einem "Opaker" wird im Rahmen der vorliegenden Erfindung eine polymerisierbaren Masse verstanden, die im wesentlichen zur farblichen Abdeckung des metallischen Untergrunds eingesetzt wird. Eine als "Opaker" einsetzbare Polymermasse wird im Hinblick auf ihre Eignung als Polymerschicht im Rahmen des vorliegenden Textes ebenfalls von dem Begriff "Polymerschicht", wie er im Zusammenhang mit den vorliegend beschriebenen Metall-Polymer-Verbunden eingesetzt wird, umfasst. Ein Metall-Polymer-Verbund, wie er im Rahmen der vorliegenden Erfindung beschrieben wird, kann daher beispielsweise auch aus einer mit Hilfe eines erfindungsgemäßen Haftvermittlers auf einer Metalloberfläche aufgebrachten Schicht eines Opakers bestehen. Geeignete Opaker sind dem Fachmann bekannt.

Gegenstand der vorliegenden Erfindung ist daher auch ein Verfahren zur Herstellung eines Metall-Polymer-Verbundes, bei dem eine nach einem erfindungsgemäßen Verfahren behandelte Metalloberfläche mit einer polymerisierbaren Masse beschichtet und die polymerisierbaren Masse anschließend polymerisiert wird.

Weiterhin ist Gegenstand der vorliegenden Erfindung ist die Verwendung eines mindestens eine Mercaptogruppe aufweisenden Alkoxysilans oder die Verwendung eines Gemischs enthaltend mindestens ein mindestens eine olefinisch ungesättigte Doppelbindung aufweisendes Alkoxysilan und mindestens ein mindestens eine Mercaptogruppe aufweisendes Alkoxysilan oder eines Kondensationsprodukts eines Gemischs aus zwei oder mehr der genannten Verbindungen als Haftvermittler für Metall-Polymer-Verbunde.

Die Erfindung wird nachfolgend durch Beispiele näher erläutert.

### Beispiele:

Folgende zweikomponentige Haftvermittler wurden hergestellt:

| Haftvermittler 1: | |
|---|---|
| Komponente A | 2 ml γ-Methacryloyloxypropyltrimethoxysilan |
| | 1 ml Trimethoxysilyl-Propanthiol |
| | 50 ml Aceton |
| Komponente B | 5 ml 50 %ige Phosphorsäure |
| | 50 ml Aceton |

| Haftvermittler 2: | |
|---|---|
| Komponente A | 2 ml γ-Methacryloyloxypropyltrimethoxysilan |
| | 1 ml Trimethoxysilyl-Propanthiol |
| | 100 ml Aceton |
| Komponente B | 10 ml Methacryloyloxyethylphosphat |
| | 100 ml Aceton |

Zur Behandlung der Metalloberflächen wurden die Oberflächen von geeigneten Metallproben (Maße: 20 * 10 * 2 mm) zunächst mit Korund gestrahlt. Anschließend erfolgte der Auftrag von Komponente A aus Haftvermittler 2. Nach dem Verdunsten des Lösemittels erfolgte der Auftrag von Komponente B aus Haftvermittler 2. In einem Zwischenschritt wurde auf die Hanvermittlerschicht ein Opaker auf Methacrylatbasis aufgetragen und polymerisiert.

Daran anschließend wurde ein Verblendkunststoff aufgetragen. Hierzu wurde auf die mit Haftvermittler und Opaker vorbehandelte Metalloberfläche ein Metallring (Durchmesser: 5 mm, Höhe: 2 mm) aufgebracht, der mit der zu polymerisierenden Masse gefüllt wurde. Anschließend wurde die Polymerisation durch Bestrahlung mit UV-Licht initiiert. Nach dem Abziehen des Metallrings befand sich ein Kunststoffzylinder mit einem Durchmesser von 5 mm und einer Höhe von 2 mm auf der behandelten Metalloberfläche.

Die so hergestellten Metall-Kunststoff-Verbunde wurden für 24 Stunden in destilliertem Wasser gelagert und anschließend in einem Druck-Scher-Versuch mit der Prüfmaschine 1435 der Fa Zwick (Vorschubgeschwindigkeit der Prüfmaschine: 1 mm min⁻¹) auf Verbundfestigkeit getestet.

Um die über Jahre erfolgende Stressbelastung der Verbunde unter Mundbedingungen zu simulieren, wurde ein Teil der Verbunde künstlich gealtert. Dies geschah entweder durch Kochen (24 Stunden) in destilliertem Wasser oder durch Temperaturwechselbelastungen (25.000 Temperaturlastwechsel, TLW, t₁ = 5 °C, t₂ = 55 °C). Die dabei erzielten Verbundfestigkeit sind in den Tabellen 1 und 2 aufgeführt.

**Tabelle 1:**

| Metall-Kunststoff-Verbund, Dentalkunststoff: Arabesk Top, Opaker: Targis-Opaker | | | | | | |
|---|---|---|---|---|---|---|
| Dentallegierung | Scherfestigkeit [Mpa] | | | | | |
| | Leerwert | | | Haftvermittler 2 | | |
| | 24 h 37 °C | 25000 TLW | 1d gekocht | 24 h 37 °C | 25000 TLW | 1d gekocht |
| Albabond | 9,9 | 4,7 | 5,4 | 28,3 | 26,4 | 29,5 |
| Mainbond EH | 12,0 | 5,3 | 4,8 | 29,7 | 27,2 | 28,4 |
| Galvanogold | 8,2 | 3,5 | 3,0 | 26,3 | 24,8 | 25,4 |
| Degunorm | 12,8 | 5,0 | 5,4 | 28,2 | 29,3 | 28,7 |
| Titan | 12,6 | 7,3 | 6,8 | 32,2 | 30,5 | 31,5 |
| Wirobond C | 10,4 | 4,4 | 5,1 | 25,8 | 24,5 | 23,6 |
| Wiron 88 | 9,1 | 4,2 | 4,9 | 26,3 | 25,2 | 25,4 |
| Aluminium (nicht gestrahlt) | 4,2 | 1,5 | 2,4 | 22,3 | 23,6 | 24,0 |
| Magnesium (nicht gestrahlt) | 3,5 | n.b. | n.b. | 21,5 | 18,3 | 17,9 |

Der Leerwert wurde an nur mit Korund gestrahlten Metalloberflächen ermittelt
n.b. = nicht bestimmt, da der Verbund zerfallen war

**Tabelle 2:**

| Metall-Kunststoff-Verbund, Metalllegierung Degunorrn, Opaker: Targis-Opaker | | | | | | |
|---|---|---|---|---|---|---|
| Dentalkunststoff | Scherfestigkeit [Mpa] | | | | | |
| | Leerwert | | | Haftvermittler 2 | | |
| | 24 h 37 °C | 25000 TLW | 1d gekocht | 24 h 37 °C | 25000 TLW | 1d gekocht |
| Sinfony | 11,4 | 5,5 | 5,3 | 27,2 | 26,5 | 25,6 |
| Art-Glass | 10,2 | 4,5 | 5,0 | 26,1 | 24,3 | 24,0 |
| Targis | 10,8 | 4,8 | 5,2 | 27,6 | 26,0 | 25,2 |
| Z 100 | 13,5 | 7,5 | 6,9 | 29,8 | 28,0 | 29,2 |
| Prodigy | 12,5 | 6,2 | 5,8 | 26,7 | 27,0 | 26,1 |
| Arabesk Top | 12,8 | 5,0 | 5,4 | 28,2 | 29,3 | 28,7 |

Zur weiteren Überprüfung der Wirksamkeit einer Oberflächenbehandlung mit einem erfindungsgemäßen Haftvermittler wurden jeweils zwei Metalloberflächen mit einem erfindungsgemäßen Haftvermittler behandelt und anschließend mit einem Dentalkunststoff verklebt. Die Behandlung der Oberfläche erfolgte analog der bereits oben beschriebenen Vorgehensweise. Die Überlappungslänge der Klebung betrug 10 mm, die Klebefläche 100 mm². Die Verklebungen wurden anschließend für eine Dauer von 24 Stunden in destilliertem Wasser gelagert und darauffolgend für fünf Tage in destilliertem Wasser gekocht.

Aus Tabelle 3 gehen die Zug-Scherfestigkeitsmeßwerte hervor, die sich aus der Verklebung von zwei Dentallegierungen ergeben.

**Tabelle 3:**

| Dentalkunststoff | Scherfestigkeit [MPa] | | | |
|---|---|---|---|---|
| | Leerwert | | Haftvermittler 2 | |
| | 24 h 37 °C | 5d gekocht | 24 h 37 °C | 5d gekocht |
| Compolute | 11,3 | 6,1 | 21,4 | 19,1 |
| Bifix | 10,8 | 5,2 | 19,3 | 17,5 |
| Multilink | 11,2 | 5,8 | 20,4 | 18,3 |
| Twinlook | 10,4 | 5,2 | 19,2 | 18,0 |

Die Druck-Scherfestigkeitsmeßwerte gemäß den Tabellen 1 und 2 zeigen, dass nach 24-stündiger Wasserlagerung bei 37 °C mit einer erfindungsgemäßen Vorbehandlung der Metalloberfläche eine deutlich höhere Verbundfestigkeit erreicht wird als an einer unbehandelten, nur mit Korund gestrahlten Oberfläche. Durch die künstliche Alterung der Verbunde, sowohl durch Kochen als auch durch Temperaturwechselbelastungen, zeigen sich die Unterschiede zwischen der behandelten und der unbehandelten Oberfläche noch deutlicher. Während die Verbundfestigkeit der unbehandelten Verbunde auf etwa 50% der Ausgangsfestigkeit abfällt, zeigte sich bei den Verbunden gemäß der vorliegenden Erfindung keine signifikante Abnahme der Verbundfestigkeit.

Die erfindungsgemäße Behandlung von sehr unterschiedlich zusammengesetzten Dentallegierungen, die Edelmetalllegierungen und nicht-Edelmetalllegierungen umfassen, macht zusätzlich deutlich, dass die haftvermittelnden Wirkungen des erfindungsgemäßen Haftvermittlers legierungsunabhängig sind.

Die im Tabelle 3 aufgeführten Verklebungen zeigen einen analoges Verhalten wie die bereits beschriebenen Verbunde. An der unbehandelten Oberfläche fallen die Messwerte durch das Kochen ebenfalls etwa auf die Hälfte der Ausgangsfestigkeit ab, während nach einer Behandlung mit einem erfindungsgemäßen Haftvermittler ein nur geringfügiger Abfall der Verbundfestigkeit auftritt.

Mit der vorliegenden Erfindung steht dabei ein Haftvermittler zur Verfügung mit dem auf Legierungsoberflächen eine haftfeste, feuchtestabile und hydrolysebeständige Verbundschicht aufgebracht werden kann, die sich für vielfältigsten Verbundkombinationen eignet und somit nicht auf die Verwendung in der Dentaltechnik beschränkt bleibt.

Eine vorteilhafte Verwendung des erfindungsgemäßen Haftvermittlers zur Herstellung von Verbundschichten ist ebenso für Lackbeschichtungen von Aluminium- und Magnesiumsblechen, im Maschinen- und Karosseriebau gegeben. Auch unter extremen Feuchtebedingungen wird damit die Haltbarkeit einer Lackbeschichtung bzw. Verklebung deutlich verbessert.

## Patentansprüche

1. Haftvermittler für Metall-Polymer-Verbundstoffe, mindestens enthaltend ein mindestens eine olefinisch ungesättigte Doppelbindung aufweisendes Alkoxysilan und mindestens ein mindestens eine Mercaptogruppe aufweisendes Alkoxysilan oder deren Kondensationsprodukte und mindestens ein organisches Lösemittel.

2. Haftvermittler gemäß Anspruch 1, **dadurch gekennzeichnet, dass** er als mindestens eine olefinisch ungesättigte Doppelbindung aufweisendes Alkoxysilan ein Alkoxysilan ausgewählt aus der Gruppe bestehend aus Vinyltrimethoxysilan, Vinyltriethoxysilan, γ-Methacryloyloxypropyltrimethoxysilan, Methacrylsäuretrimethylsilyloxyethylester, Methacrylsäure-tris-trimethylsilyloxy-silyl-propylester, Trimethoxysilylpropanthiol oder Triethoxysilylpropanthiol oder ein Gemisch aus zwei oder mehr davon oder ein Kondensationsprodukt aus zwei oder mehr davon enthält.

3. Haftvermittler gemäß Anspruch 1 oder 2, **dadurch gekennzeichnet, dass** er mindestens eine Säure oder ein saures Monomeres oder ein Gemisch aus zwei oder mehr davon enthält.

4. Haftvermittler gemäß einem der Ansprüche 1 bis 3, **dadurch gekennzeichnet, dass** er mindestens eine organische Säure mit mindestens einer olefinisch ungesättigten Doppelbindung enthält.

5. Haftvermittler gemäß einem der Ansprüche 1 bis 4, **dadurch gekennzeichnet, dass** er als organisches Lösemittel ein Lösemittel mit einem Siedepunkt von weniger als 100 °C enthält.

6. Haftvermittler, mindestens umfassend zwei getrennt vorliegende Komponenten A und B, wobei
a) Komponente A mindestens ein mindestens eine olefinisch ungesättigte Doppelbindung aufweisendes Alkoxysilan und mindestens ein mindestens eine Mercaptogruppe aufweisendes Alkoxysilan oder deren Kondensationsprodukte und mindestens ein Lösemittel und
b) Komponente B mindestens eine Säure oder mindestens ein saures Monomeres oder ein Gemisch aus zwei oder mehr davon und mindestens ein Lösemittel enthält.

7. Haftvermittler gemäß Anspruch 6, **dadurch gekennzeichnet, dass** Komponente A oder Komponente B oder beide ein Lösemittel oder Lösemittelgemisch enthalten.

8. Haftvermittler gemäß einem der Ansprüche 6 oder 7, **dadurch gekennzeichnet, dass** Komponente A
c) 0,5 bis 30 Gew.-% mindestens eines Alkoxysilans mit einer olefinisch ungesättigten Doppelbindung
d) 0,5 bis 20 Gew.-% mindestens eines Alkoxysilans mit mindestens einer Mercaptogruppe und
e) 50 bis 99 Gew.-% eines Lösemittels oder eines Lösemittelgemischs
enthält.

9. Haftvermittler gemäß einem der Ansprüche 6 bis 8, **dadurch gekennzeichnet, dass** Komponente B
f) 0,5 bis 50 Gew.-%, einer Säure oder eines sauren Monomeren und
g) 50 bis 99,5 Gew.-% eines Lösemittels oder eines Lösemittelgemischs
enthält.

10. Verfahren zur Behandlung einer Metalloberfläche, bei dem eine Metalloberfläche mit einem Haftvermittler nach einem der Ansprüche 1 bis 9 versehen wird.

11. Verfahren nach Anspruch 10, **dadurch gekennzeichnet, dass** ein Haftvermittler nach einem der Ansprüche 6 bis 9 eingesetzt wird.

12. Verfahren nach Anspruch 11, **dadurch gekennzeichnet, dass** die Metalloberfläche zuerst mit Komponente A und anschließend mit Komponente B behandelt wird.

13. Verfahren zur Herstellung eines Metall-Polymer-Verbundes, bei dem eine gemäß einem Verfahren nach einem der Ansprüche 10 bis 12 behandelte Metalloberfläche mit einer polymerisierbaren Masse beschichtet und die polymerisierbare Masse anschließend polymerisiert wird.

14. Verwendung eines mindestens eine Mercaptogruppe aufweisenden Alkoxysilans oder die Verwendung eines Gemischs enthaltend mindestens ein mindestens eine olefinisch ungesättigte Doppelbindung aufweisendes Alkoxysilan und mindestens ein mindestens eine Mercaptogruppe aufweisendes Alkoxysilan oder eines Kondensationsprodukts eines Gemischs aus zwei oder mehr der genannten Verbindungen als Haftvermittler für Metall-Polymer-Verbunde.

## Claims

1. Adhesive for metal-polymer composites, at least comprising an alkoxysilane having at least one olefinic unsaturated double bond and at least one alkoxysilane having at least one mercapto group or condensation products thereof and at least one organic solvent.

2. Adhesive according to claim 1, **characterised in that** it comprises as alkoxysilane having at least one olefinic unsaturated double bond an alkoxysilane selected from the group comprising vinyltrimethoxysilane, vinyltriethoxysilane, γ-methacryloyloxypropyltrimethoxysilane, methacrylic acid trimethylsilyloxyethyl ester, methacrylic acid tris-trimethylsilyloxysilylpropyl ester, trimethoxysilylpropanethiol or triethoxysilylpropanethiol or a mixture of two or more thereof or a condensation product of two or more thereof.

3. Adhesive according to claim 1 or claim 2, **characterised in that** it comprises at least one acid or an acidic monomer or a mixture of two or more thereof.

4. Adhesive according to one of claims 1 to 3, **characterised in that** it comprises at least one organic acid with at least one olefinic unsaturated double bond.

5. Adhesive according to one of claims 1 to 4, **characterised in that** it comprises as organic solvent a solvent with a boiling point of less than 100 °C.

6. Adhesive, comprising at least two separately present components A and B, wherein
a) component A comprises at least one alkoxysilane having at least one olefinic unsaturated double bond and at least one alkoxysilane having at least one mercapto group or condensation products thereof and at least one solvent and
b) component B comprises at least one acid or at least one acidic monomer or a mixture of two or more thereof and at least one solvent.

7. Adhesive according to claim 6, **characterised in that** component A or component B or both comprise a solvent or a mixture of solvents.

8. Adhesive according to one of claims 6 or 7, **characterised in that** component A comprises
c) 0.5 to 30 wt.% of at least one alkoxysilane with an olefinic unsaturated double bond
d) 0.5 to 20 wt.% of at least one alkoxysilane with at least one mercapto group and
e) 50 to 99 wt.% of a solvent or a mixture of solvents.

9. Adhesive according to one of claims 6 to 8, **characterised in that** component B comprises
f) 0.5 to 50 wt.% of an acid or an acidic monomer and
g) 50 to 99.5 wt.% of a solvent or a mixture of solvents.

10. Process for treating a metal surface, wherein a metal surface is provided with an adhesive according to one of claims 1 to 9.

11. Process according to claim 10, **characterised in that** an adhesive according to one of claims 6 to 9 is used.

12. Process according to claim 11, **characterised in that** the metal surface is treated first with component A and then with component B.

13. Process for producing a metal-polymer composite, wherein a metal surface treated according to a process according to one of claims 10 to 12 is coated with a polymerisable mass and the polymerisable mass is then polymerised.

14. Use of an alkoxysilane having at least one mercapto group or use of a mixture comprising at least one alkoxysilane having at least on olefinic unsaturated double bond and at least one alkoxysilane having at least one mercapto group or a condensation product of a mixture of two or more of the compounds cited as adhesive for metal-polymer composites.

## Revendications

1. Agent adhésif pour matériaux composites métal-polymère, contenant au moins un alcoxysilane ayant au moins une liaison double à insaturation oléfine et au moins un alcoxysilane présentant au moins un groupe thiol ou leurs produits de condensation et au moins un solvant organique.

2. Agent adhésif selon la revendication 1, **caractérisé en ce qu'**il contient comme alcoxysilane ayant au moins une liaison double à insaturation oléfine un alcoxysilane choisi parmi le groupe comprenant le vinyltrimethoxysilane, le vinyltriethoxysilane, le γ-methacryloyloxypropyltrimethoxysilane, le trimethylsilyloxyethylester d'acide méthacrylique, le tris-trimethylsilyloxy-silylpropylester d'acide méthacrylique, le trimethoxysilylpropanethiol ou le triethoxysilylpropanethiol ou un mélange de deux ou plus ou un produit de condensation de deux ou plus.

3. Agent adhésif selon la revendication 1 ou 2, **caractérisé en ce qu'**il contient au moins un acide ou un monomère acide ou un mélange de deux ou plus.

4. Agent adhésif selon l'une des revendications 1 à 3, **caractérisé en ce qu'**il contient au moins un acide organique avec au moins une liaison double à insaturation oléfine.

5. Agent adhésif selon l'une des revendications 1 à 4, **caractérisé en ce qu'**il contient comme solvant organique un solvant avec un point d'ébullition de moins de 100°C.

6. Agent adhésif comprenant au moins deux composantes A et B qui sont présentes séparément, dans quel cas
a) la composante A contient au moins un alcoxysilane ayant au moins une liaison double à insaturation oléfine et au moins un alcoxysilane présentant au moins un groupe thiol ou leurs produits de condensation et au moins un solvant et
b) la composante B contient au moins un acide ou au moins un monomère acide ou un mélange de deux ou plus et au moins un solvant.

7. Agent adhésif selon la revendication 6, **caractérisé en ce que** la composante A ou la composante B ou les deux contiennent un solvant ou un mélange de solvants.

8. Agent adhésif selon l'une des revendications 6 ou 7, **caractérisé en ce que** la composante A contient
c) 0,5 à 30 % en poids d'au moins un alcoxysilane avec une liaison double à insaturation oléfine
d) 0,5 à 20 % en poids d'au moins un alcoxysilane avec au moins un groupe thiol et
e) 50 bis 99 % en poids d'un solvant ou d'un mélange de solvants.

9. Agent adhésif selon l'une des revendications 6 à 8, **caractérisé en ce que** la composante B contient
f) 0,5 bis 50 % en poids d'un acide ou d'un monomère acide et
g) 50 à 99,5 % en poids d'un solvant ou d'un mélange de solvants.

10. Procédé destiné au traitement d'une surface de métal, dans lequel une surface de métal est pourvue d'un agent adhésif selon l'une des revendications 1 à 9.

11. Procédé selon la revendication 10, **caractérisé en ce qu'**un agent adhésif est utilisé selon l'une des revendications 6 à 9.

12. Procédé selon la revendication 11, **caractérisé en ce que** la surface de métal est d'abord traitée avec la composante A et ensuite avec la composante B.

13. Procédé destiné à la fabrication d'un composite métal-polymère, dans lequel une surface de métal traitée conformément à un procédé selon l'une des revendications 10 à 12 est revêtue d'une masse polymérisable et la masse polymérisable est ensuite polymérisée.

14. Utilisation d'un alcoxysilane ayant au moins un groupe thiol ou utilisation d'un mélange contenant au moins un alcoxysilane ayant au moins une liaison double à insaturation oléfine et au moins un alcoxysilane ayant au moins un groupe thiol ou d'un produit de condensation d'un mélange de deux ou plus des composés précités en tant qu'agent adhésif pour des composites métal-polymère.
